(19) Europäisches Patentamt
European Patent Office
Office européen des brevets

(11) **EP 4 089 330 B1**

(12) **EUROPÄISCHE PATENTSCHRIFT**

(45) Veröffentlichungstag und Bekanntmachung des Hinweises auf die Patenterteilung:
**23.10.2024   Patentblatt 2024/43**

(21) Anmeldenummer: **22160870.6**

(22) Anmeldetag: **08.03.2022**

(51) Internationale Patentklassifikation (IPC):
**F23N 5/26** *(2006.01)*      **G01N 33/22** *(2006.01)*
**F17D 1/04** *(2006.01)*

(52) Gemeinsame Patentklassifikation (CPC):
**F23N 5/265; F17D 1/04;** F23N 2221/10;
G01N 33/225

(54) **VERFAHREN UND SYSTEM ZUR BESTIMMUNG EINES BRENNWERTS EINES BRENNGASES IN EINEM GASNETZABSCHNITT UND DESSEN VERWENDUNG**

METHOD AND SYSTEM FOR DETERMINING THE CALORIFIC VALUE OF A FUEL GAS IN A GAS NETWORK SECTION AND ITS USE

PROCÉDÉ ET SYSTÈME DE DÉTERMINATION D'UN POUVOIR CALORIFIQUE D'UN GAZ COMBUSTIBLE DANS UNE SECTION DE RÉSEAU DE GAZ ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.05.2021   DE 102021204885**

(43) Veröffentlichungstag der Anmeldung:
**16.11.2022   Patentblatt 2022/46**

(73) Patentinhaber: **Viessmann Climate Solutions SE 35108 Allendorf (DE)**

(72) Erfinder:
• **ENGEL, Uwe**
**35099 Burgwald (DE)**

• **IMHOF, Michael**
**35066 Frankenberg (DE)**
• **VOGT, Arno**
**35099 Burgwald (DE)**
• **SCHEER, Benjamin**
**59969 Hallenberg (DE)**

(74) Vertreter: **MERH-IP Matias Erny Reichl Hoffmann Patentanwälte PartG mbB Paul-Heyse-Strasse 29 80336 München (DE)**

(56) Entgegenhaltungen:
WO-A1-2004/065915      WO-A1-2011/096799
DE-A1- 102019 115 973      DE-A1- 19 736 528
US-A1- 2015 355 119

**Beschreibung**

TECHNISCHES FELD

Die Erfindung betrifft ein Verfahren und ein System zur Bestimmung eines Brennwerts eines Brenngases in einem Gasnetzabschnitt zur Verwendung für eine Gasabrechnung von Verbrauchern in dem Gasnetzabschnitt, wobei der Brennwert des Brenngases für den Gasnetzabschnitt sicher und verlässlich ermittelt werden kann und der ermittelte Wert für die Gasabrechnung der Verbraucher in dem Gasnetzabschnitt verwendet werden kann.

TECHNISCHER HINTERGRUND

Eine Aufgabe der Erfindung ist es, ein Verfahren und ein System bereitzustellen, welches ermöglicht, einen Brennwert eines Brenngases in einem Gasnetzabschnitt auf einfache Weise sicher und verlässlich zu bestimmen, so dass der ermittelte Wert für eine Gasabrechnung der Verbraucher in dem Gasnetzabschnitt verwendet werden kann.

Der Brennwert eines in einem Gasnetzabschnitt geführten Brenngases hängt stark von der Zusammensetzung des Brenngases ab. Zusatzgase können beispielsweise je nach ihrer Art und ihrem Anteil die Beschaffenheit und damit den Brennwert des im Gasnetz geführten Grundgases stark beeinflussen und damit auch den Betrieb bzw. den Verbrauch von Geräten, d.h. Verbrauchern, wie beispielsweise Gasbrennern oder Gasheizkesseln. Der Anteil eines Zusatzgases im Brenngas kann dabei in Gasnetzabschnitten sowohl regional spezifisch, als auch zeitabhängig stark schwanken und somit auch den Brennwert des Brenngases regional und zeitabhängig stark beeinflussen.

Motiviert durch eine langfristige Einsparung an $CO_2$-Emissionen, ist beispielsweise die Einspeisung klimaneutral erzeugten Wasserstoffs als Zusatzgas im bestehenden Erdgasnetz mit Volumenanteilen von bis zu 40 Vol.-% geplant. Die Brenneigenschaft von Erdgas und Wasserstoff unterscheiden sich jedoch deutlich.

Vor diesem Hintergrund ist die Kenntnis des Brennwerts eines in einem Gasnetzabschnitt geführten Brenngases, insbesondere auch zu unterschiedlichen Zeitpunkten, wünschenswert.

Die EP 2 450 704 B1 beschreibt beispielsweise ein Verfahren zur Bestimmung des Brennwertes von Brenngas, insbesondere von Erdgas, in Gasnetzen, insbesondere in Regional- oder Verteilnetzen mit mindestens zwei Einspeisestellen, einer Mehrzahl von Netzknoten und mehreren Ausspeisestellen, wobei a) die Brennwerte und Mengen an den Einspeisestellen des Gasnetzes gemessen werden, b) die Mengen an den Ausspeisestellen des Gasnetzes auf Basis von Lastprofilen gemäß entsprechender Gleichungen geschätzt und summiert werden, c) die jeweils ermittelten Werte zusammen mit topologischen Daten des Gasnetzes einer Auswerteeinheit zugeführt und die Brennwerte an mindestens einer Ausspeisestelle rechnerisch bestimmt werden.

Die EP 3 287 750 B1 beschreibt beispielsweise ein Gaszählersystem und ein Heizwertschätzverfahren. Das Gaszählersystem ist dazu eingerichtet, einen Heizwert eines Gases zu schätzen, das einen ersten Gaszähler passiert, und einen Heizwert eines Gases zu schätzen, das einen zweiten Gaszähler passiert, der getrennt von dem ersten Gaszähler bereitgestellt ist. Die Schätzung basiert auf dem Heizwert des Gases des ersten Gaszählers, welcher in Bezug auf den zweiten Gaszähler entlang einer Gasversorgungsleitung in einem vorbestimmten Abstand angeordnet ist.

Die DE 10 2018 106 576 A1 beschreibt beispielsweise ein Verfahren zum Bestimmen eines Gasverbrauchs bei wechselnder Gasbeschaffenheit in einem Gasnetz, mit wenigstens einer Zählereinrichtung zur Erfassung zumindest eines Gasvolumenstroms, wobei eine oder mehrere lokale Gaseinspeisungspunkte im Gasnetz vorgesehen sind. Zumindest eine aktuelle Gasbeschaffenheit wird in einem Netzabschnitt des Gasnetzes bestimmt, wobei wenigstens eine Verbrauchsstelle im Netzabschnitt einen Gasvolumenstrom entnimmt. Zumindest die aktuelle Gasbeschaffenheit des Gases im Netzabschnitt wird an eine Datenzentrale übermittelt. Ein Verbrauch an Gasvolumenstrom der wenigstens einen Verbrauchsstelle wird mit deren Zählereinrichtung bestimmt. Der aktuelle Verbrauch wird anhand zeitgenauer Zählermesswerte der Zählereinrichtung an die Datenzentrale übermittelt. Der aktuelle Brennwert wird anhand der Gasbeschaffenheit im Netzabschnitt bestimmt und zumindest die zeitgenauen Brennwerte werden mit den Zählermesswerten der Verbrauchsstelle verknüpft.

Und die FR 3 030 034 A1 beschreibt beispielsweise einen Gaszähler zur Kundeninstallation eines Gasverteilungsnetzes, umfassend eine Leitung mit einer Gaseinlassöffnung und einer Gasauslassöffnung, eine Einheit zum Messen einer in der Leitung zirkulierenden Gasmenge, eine Analyseeinheit für das in dem Kanal strömende Gas, um zumindest eine Teilzusammensetzung davon zu bestimmen, und eine Datenübertragungseinheit, die mit der Analyseeinheit verbunden und angeordnet ist, um eine Kennung und Analysedaten zu übertragen, und mindestens eine vom Messgerät getrennte Datenerfassungseinheit.

Die DE 10 2019 115973 A1 zeigt eine Messeinrichtung zur Bestimmung des Brennwerts eines Gasstroms in einer Gasleitung, insbesondere eines Gasverteilnetzes, mit einem Brennwert-Sensor, der dazu eingerichtet ist, einen Wert für den spezifischen Gasbrennwert oder für die Wobbezahl eines Gases in einer Gasleitung zu messen, und mit einer Steuereinrichtung, die dazu eingerichtet ist, aus dem gemessenen Wert für den spezifischen Gasbrennwert oder die Wobbezahl und optional weiteren erhaltenen Messwerten über den Gasstrom einen Wert für den Brennwert des Gasstroms zu bestimmen. Die Erfindung betrifft weiter ein Gasverteilnetz mit einer solchen Messeinrichtung.

Die WO 2004/065915 A1 zeigt ein Verfahren und eine Vorrichtung zur genaueren Messung einer Gasversorgung mittels eines Gaszählers. Dazu wird ein verbrauchsgewichteter Korrekturfaktor durch gewichtete Mittelung eines Sensorfehlerfaktors des Gaszählers mit einem für den Ort der Gasversorgung charakteristischen Verbraucherprofil ermittelt und das Messsignal mit dem Korrekturfaktor in einen korrigierten Verbrauchs- oder Leistungswert umgerechnet. Ausführungsbeispiele sind u.a.: Betrieb des Gaszählers als Volumen-, Masse- oder Energiezähler; Formulierung zur Ermittlung des Korrekturfaktors mit Sensorfehlerfaktoren und volumen-, masse- oder energiebezogenen Verbrauchsprofilen sowie Messsignalkorrektur bei nicht registrierenden oder registrierenden Gaszählern.

LÖSUNG DES PROBLEMS

Die oben genannte Aufgabe wird mit den Merkmalen der unabhängigen Ansprüche gelöst. Die abhängigen Ansprüche sind auf besondere Ausführungsformen der Erfindung gerichtet. Im Folgenden werden Aspekte und Ausführungsbeispiele erläutert, die dem Verständnis der Erfindung dienen.

Ein Verfahren zur Bestimmung eines Brennwerts eines Brenngases in einem Gasnetzabschnitt zur Verwendung für eine Gasabrechnung von Verbrauchern in dem Gasnetzabschnitt umfasst den Schritt a) des Bestimmens eines Brennwerts eines Brenngases durch mindestens einen Verbraucher in dem Gasnetzabschnitt.

Der hierin verwendete Begriff Verbraucher bezieht sich auf den Verbrauch von Brenngas aus einem Gasnetzabschnitt. Verbraucher können daher beispielsweise Gasbrenner, Gasheizkessel oder Brennstoffzellenmodule sein. Im Folgenden wird ebenfalls der Begriff "Gerät" synonym für den Begriff "Verbraucher" verwendet.

Der hierin verwendete Begriff Brenngas bezieht sich auf das in einem Gasnetzabschnitt geführte Brenngas. Es kann sich also beispielsweise um Erdgas als Grundgas handeln, aber auch um anteilige Mischungen von Erdgas und einem oder mehreren Zusatzgasen, wie beispielsweise Wasserstoff, Stickstoff, Methan, Schwefel, Kohlenstoffmonoxid, Kohlenstoffdioxid oder Propan.

Das Bestimmen des Brennwerts in Schritt a) erfolgt durch den mindestens einen Verbraucher anhand von Regelungsparametern. Der Betriebszustand, in welchem der Brennwert durch den mindestens einen Verbraucher bestimmt wird, ist dabei nicht eingeschränkt. Die Methode zur Bestimmung des Brennwerts ist ebenfalls nicht eingeschränkt und wird sich nach der Art und Ausgestaltung des mindestens einen Verbrauchers richten.

In einer ersten erfindungsgemäßen Ausführungsform ist der mindestens eine Verbraucher ein Gasheizkessel und in Schritt a) wird der Brennwert des Brenngases durch den mindestens einen Verbraucher anhand eines Zusammenhangs zwischen Modulationsgrad, Wärmebelastung und Luftmassenstrom bestimmt.

Das Bestimmen des Brennwerts kann mit anderen Worten auch als eine Bilanzierung einer Brennstoffenergie bezeichnet werden. So kann beispielsweise mittels Auswertung von internen Datenpunkten die Brennstoffenergie Q [kWh] wie folgt berechnet werden:

Basis für die Berechnung ist der Modulationsgrad [%]. Durch Kennfelder innerhalb eines Steuerungsgeräts, beispielsweise eines Gasfeuerungsautomaten (GFA), ergibt sich ein linearer Zusammenhang zwischen Modulationsgrad [%], Wärmebelastung [kW] und Luftmassenstrom [g/s]. Die Brennstoffenergie [kWh] berechnet sich durch Integration der Modulation [%] und damit der Wärmebelastung [kW] über die Zeit.

Der Modulationsgrad wird beispielsweise durch einen Kesselregler oder einen Trinkwasserregler vorgegeben. Daraus erfolgt eine Sollwertvorgabe für den Luftmassenstrom [g/s] und für den IO-Stromsollwert [Pkt.]. Der lineare Zusammenhang zwischen Modulationsgrad und Wärmebelastung gilt nur, solange das Gerät bei Nenn-Luftmassenstrom [g/s] und Nenn-Lambda [-] betrieben wird. Die Führungsgröße für Lambda ist eine IO-Strom basierte Regelung. Auf Basis von Erfahrungswerten gibt es unter normalen Betriebsbedingungen eine Lambda-Streuung +/- 1 Lambda-Zehntel. Diese Streuungen resultieren u.a. aus Alterungserscheinungen der IO-Elektrode und anderen Einflussgrößen. Infolgedessen ergibt sich eine Toleranz für die berechnete Brennstoffenergie [kWh] in Höhe von ca. +/- 2%.

In einer zweiten erfindungsgemäßen Ausführungsform ist der mindestens eine Verbraucher ebenfalls ein Gasheizkessel und in Schritt a) wird der Brennwert des Brenngases durch den mindestens einen Verbraucher anhand eines Zusammenhangs zwischen Wärmeleistung und Wirkungsgrad bestimmt.

Wie bereits dargelegt, kann das Bestimmen des Brennwerts mit anderen Worten auch als eine Bilanzierung einer Brennstoffenergie bezeichnet werden.

So kann beispielsweise die Bilanzierung durch Ermittlung der Wärmeleistung QH [kW] und Berechnung des Wirkungsgrads η über den folgenden Zusammenhang erfolgen:

$$\eta_{hs} = \frac{output}{input} = \frac{m \times cp \times dT}{V_{Gas} \times GCV} \tag{1}$$

wobei:

m = Massenstrom Wärmeträgermedium $\left[\frac{kg}{h}\right]$

cp = spezifische Wärmekapazität Wärmeträgermedium $\left[\frac{kJ}{kg} * K\right]$

dT = Temperaturdifferenz Wärmeträgermedium [K]

$\dot{V}_{Norm\_Gas}$ = Norm Volumenstrom Brenngas $\left[m^3/h\right]$

$GCV \overset{\wedge}{=} Hs = Brennwert$, gross calorific value $\left[kJ/m^3\right]$

Die Messung der Wärmeleistung erfolgt durch einen internen Volumenstromsensor sowie der Vorlauftemperatur (Kesseltemperatur) und der Rücklauftemperatur (interne Temperatur im Volumenstrom-sensor). Die Dichte sowie die spezifische Wärmekapazität werden durch Stütztabellen hinterlegt.

Die Berechnung des Wirkungsgrads η erfolgt analog dem Verfahren des feuerungstechnischen Wirkungsgrades. Ausgehend von maximal 100% Wirkungsgrad wird der Abgasverlust und der Strahlungsverlust abgezogen.

$$\eta_{hs} = 100 - qa,hs - qs \qquad (2)$$

Der Abgasverlust qA,hs ist die Summe aus sensiblen und latenten Wärmeinhalt, der nicht genutzt wird. Für die Berechnung des sensiblen Anteils wird die Abgastemperatur genutzt. Zudem wird der Abgasmassenstrom auf Basis des Modulationsgrades und dem Nenn-Lambda mit den Toleranzen wie oben dargelegt berechnet.

Der latente Anteil des Abgasverlusts beschreibt den Energieeinhalt, der nicht durch Kondensation dem Output in Form von Wärme gutgeschrieben wird. Eine direkte Messung dieses latenten Anteils ist nicht möglich, daher bedient sich das Verfahren eines Zusammenhangs zwischen der mittleren Temperatur im Wärmeübertrager, dem Modulationsgrad und einem hinterlegten Kennfeld für den Kondensationsgrad.

Zur Quantifizierung des Strahlungsverlusts qs wird die Kesseltemperatur und der Modulationsgrad verwendet.

Ermittlung Brennwert hs

$$output = V_{Norm} * hs$$

$$hs = output / V_{Norm}$$

$V_{Norm}$ ableiten aus Ansteuerung vom Gasventil

Der Brennwert ist grundsätzlich von den Eigenschaften des in einem Gasnetzabschnitt geführten Brenngases abhängig. So kann beispielsweise ein dem Erdgas als Grundgas anteilig hinzugefügtes Zusatzgas den Brennwert bereits stark beeinflussen. Die Art des Zusatzgases ist dabei nicht eingeschränkt. Das Zusatzgas kann beispielsweise ausgewählt sein aus einer Gruppe bestehend aus Wasserstoff, Stickstoff, Methan, Schwefel, Kohlenstoffmonoxid, Kohlenstoffdioxid und Propan.

Der Anteil eines Zusatzgases im Brenngas in Gasnetzabschnitten kann nicht nur regional spezifisch, sondern auch zeitabhängig stark schwanken und somit auch den Brennwert des Brenngases regional und zeitabhängig stark beeinflussen.

In einer bevorzugten Ausführungsform kann daher Schritt a) des Verfahrens mindestens einmal innerhalb einer vorgegebenen Zeitspanne Δt durchgeführt werden. Die vorgegebene Zeitspanne Δt ist nicht eingeschränkt und kann sich an Erfahrungswerten in Bezug auf die zeitlichen Schwankungen der Gaszusammensetzung im jeweiligen Gasnetzabschnitt beziehen. Auf diese Weise ist aber jedenfalls eine sichere, verlässliche und zeitgenaue Ermittlung des Brennwerts des Brenngases im Gasnetzabschnitt möglich.

In einer weiteren bevorzugten Ausführungsform kann Schritt a) des Verfahrens zwei oder mehrmals innerhalb der vorgegebenen Zeitspanne Δt durchgeführt werden. Mittels der Bestimmung des Brennwerts des Brenngases durch den mindestens einen Verbraucher zu zwei oder mehr Zeitpunkten innerhalb der vorgegebenen Zeitspanne $\Delta t$ kann zusätzlich die Genauigkeit und die Verlässlichkeit des ermittelten Brennwerts des Brenngases im Gasnetzabschnitt verbessert werden.

Das Verfahren umfasst weiterhin den Schritt b) des Übermittelns des in Schritt a) bestimmten Brennwerts und von Positionsdaten des mindestens einen Verbrauchers an eine zentrale Einheit.

In einer bevorzugten Ausführungsform kann die Übermittlung des in Schritt a) bestimmten Brennwerts und der Positionsdaten des mindestens einen Verbrauchers in Echtzeit an die zentrale Einheit erfolgen. Der Begriff Echtzeit ist hierin breit auszulegen. Echtzeit bedeutet hierin die zeitliche Verzögerung, die zwischen der Übermittlung des Brennwerts durch den mindestens einen Verbraucher an die zentrale Einheit und des Ausgebens des ermittelten Brennwerts durch die zentrale Einheit liegt.

In einer weiteren bevorzugten Ausführungsform können in Schritt a) zusätzlich Informationen über einen Bestimmungszeitpunkt des Brennwerts des Brenngases durch den mindestens einen Verbraucher gespeichert werden und in Schritt b) an die zentrale Einheit übermittelt werden. Ein Bestimmungszeitpunkt kann beispielsweise eine Uhrzeit sein. Da eine Gasabrechnung im Allgemeinen zu einem späteren Zeitpunkt als die Bestimmung des Brennwerts im Gasnetzabschnitt erfolgt, kann auf diese Weise eine zeitgenaue Zuordnung des ermittelten Brennwerts im Gasnetzabschnitt erfolgen. Bevorzugt können die durch den mindestens einen Verbraucher zu den zwei oder mehr Zeitpunkten bestimmten Brennwerte des Brenngases dann gesammelt und in Schritt b) mit den Positionsdaten des mindestens einen Verbrauchers und optional den Informationen über den Bestimmungszeitpunkt an die zentrale Einheit übermittelt werden.

Zentrale Einheit bedeutet hierin eine Einheit zur Übermittlung (senden und empfangen), Speicherung und Verarbeitung von Daten. Eine zentrale Einheit kann also beispielsweise eine Leitwarte, ein Server oder eine Cloud sein. Verbraucher, aber zusätzlich auch Erzeuger, in einem Gasnetzabschnitt können dabei vorteilhaft mit der zentralen Einheit ein Netzwerk bilden. Alternativ, oder zusätzlich hierzu kann eine zentrale Einheit auch mit mehreren Netzwerken oder auch Clustern von Verbrauchern und/oder Erzeugern verbunden sein. Die Netzwerke oder Cluster können dabei beispielsweise unterschiedlichen, regional spezifischen Gasnetzabschnitten zugeordnet sein.

Zusätzlich zu dem in Schritt a) bestimmten Brennwert werden in Schritt b) des Verfahrens auch Positionsdaten des mindestens einen Verbrauchers an die zentrale Einheit übermittelt.

In einer bevorzugten Ausführungsform können die Positionsdaten anonymisiert sein. Anonymisiert kann beispielsweise bedeuten, dass lediglich die Zuordnung eines Gasanschlusses zu einem Bereich des Gasnetzabschnitts als Positionsdaten übermittelt werden.

In einer weiteren bevorzugten Ausführungsform kann in Schritt a) der Brennwert des Brenngases jeweils durch zwei oder mehr Verbraucher bestimmt werden, wobei in Schritt b) die in Schritt a) bestimmten Brennwerte und die Positionsdaten der zwei oder mehr Verbraucher an die zentrale Einheit übermittelt werden können und in Schritt c) ein gemittelter Brennwert des Brenngases in dem Gasnetzabschnitt durch die zentrale Einheit bestimmt werden kann. Durch die Vielzahl an Verbrauchern/Geräten im Feld kann auf diese Weise ebenfalls die Sicherheit und Verlässlichkeit des in Schritt c) ermittelten Brennwerts des Brenngases im Gasnetzabschnitt erhöht werden.

In einer besonders bevorzugten Ausführungsform kann die Übermittlung der in Schritt a) bestimmten Brennwerte und der Positionsdaten der zwei oder mehr Verbraucher an die zentrale Einheit in Echtzeit erfolgen.

In einer weiteren bevorzugten Ausführungsform können in Schritt a) zusätzlich jeweils Informationen über den Bestimmungszeitpunkt des Brennwerts des Brenngases durch die zwei oder mehr Verbraucher gespeichert werden und in Schritt b) an die zentrale Einheit übermittelt werden.

Alternativ oder zusätzlich zur Mittelung der Brennwerte, um den gemittelten Brennwert des Brenngases zu bestimmen, können bei einer Vielzahl von durch Verbraucher bestimmten Brennwerten überdies statistische Verfahren in der zentralen Einheit zur Anwendung kommen, um den Brennwert des Brenngases in dem Gasnetzabschnitt zu bestimmen. So können beispielsweise Ausreißer in den durch die Verbraucher bestimmten Brennwerten nicht berücksichtigt oder auch gewichtete Mittelungen durch die zentrale Einheit durchgeführt werden. Dies kann die Genauigkeit des ermittelten Brennwerts des Brenngases im Gasnetzabschnitt weiter erhöhen.

Überdies kann gemäß einer bevorzugten Ausführungsform in Schritt a) der Brennwert des Brenngases durch die zwei oder mehr Verbraucher jeweils zeitgleich bestimmt werden.

Alternativ kann gemäß einer weiteren bevorzugten Ausführungsform in Schritt a) der Brennwert des Brenngases durch die zwei oder mehr Verbraucher jeweils zeitversetzt bestimmt werden.

Zeitversetzt bedeutet in diesem Zusammenhang, dass ein erster Verbraucher an einem, oder auch mehreren, ersten Zeitpunkten den Brennwert des Brenngases bestimmen kann, wohingegen ein zweiter Verbraucher an einem, oder auch mehreren, zu den ersten Zeitpunkten zeitversetzten zweiten Zeitpunkten den Brennwert des Brenngases bestimmen kann. Das Intervall, um welches die Bestimmung des Brennwerts des Brenngases zeitversetzt erfolgt, ist nicht eingeschränkt und wird sich nach der Gesamtzahl der Verbraucher richten. Das Intervall kann aber beispielsweise 30 min. betragen.

Das Verfahren umfasst weiterhin den Schritt c) des Bestimmens des Brennwerts des Brenngases in dem Gasnetzabschnitt durch die zentrale Einheit basierend auf dem übermittelten Brennwert des mindestens einen Verbrauchers und den Positionsdaten.

In einer bevorzugten Ausführungsform können in der zentralen Einheit zusätzlich Informationen über eine Beschaffenheit des Gasnetzabschnitts gespeichert sein. Dann kann in Schritt c) das Bestimmen des Brennwerts des Brenngases im Gasnetzabschnitt unter Berücksichtigung der Informationen über die Beschaffenheit des Gasnetzabschnitts erfolgen. Informationen über die Beschaffenheit des Gasnetzabschnitts können beispielsweise Informationen über die Gruppierung von Geräten, d.h. Verbrauchern in dem Gasnetzabschnitt sein.

Das Verfahren umfasst weiterhin den Schritt d) des Ausgebens des Brennwerts des Brenngases in dem Gasnetzabschnitt durch die zentrale Einheit zur Verwendung für eine Gasabrechnung von Verbrauchern in dem Gasnetzabschnitt.

Ein System zur Bestimmung eines Brennwerts eines Brenngases in einem Gasnetzabschnitt zur Verwendung für eine Gasabrechnung von Verbrauchern in dem Gasnetzabschnitt umfasst einen Gasnetzabschnitt, in welchem mindestens ein Verbraucher angeordnet ist, wobei der mindestens eine Verbraucher eine Einrichtung zur Übermittlung von Daten aufweist und eingerichtet ist, einen Brennwert eines Brenngases zu bestimmen. Hierzu kann der mindestens eine Verbraucher beispielsweise eine Steuereinrichtung aufweisen. Das System umfasst außerdem eine zentrale Einheit.

In einer bevorzugten Ausführungsform des Systems kann der mindestens eine Verbraucher zusätzlich eine Zeitmessvorrichtung aufweisen.

In einer weiteren bevorzugten Ausführungsform des Systems können in dem Gasnetzabschnitt zwei oder mehr Verbraucher angeordnet sein, wobei die zwei oder mehr Verbraucher jeweils eine Einrichtung zur Übermittlung von Daten aufweisen und jeweils eingerichtet sind, einen Brennwert eines Brenngases zu bestimmen. Die zwei oder mehr Verbraucher können weiterhin jeweils eine Zeitmessvorrichtung aufweisen.

KURZE BESCHREIBUNG DER FIGUREN

Figuren 1 bis 4 zeigen schematisch Ausführungsformen des Verfahrens, sowie Ausführungsformen des Systems.

Figur 1 zeigt ein Flussdiagramm einer Ausführungsform eines Verfahrens zur Bestimmung eines Brennwerts eines Brenngases in einem Gasnetzabschnitt zur Verwendung für eine Gasabrechnung von Verbrauchern in dem Gasnetzabschnitt.

Figur 2 zeigt ein Blockdiagramm zur Illustration einer Ausführungsform eines Systems zur Bestimmung eines Brennwerts eines Brenngases in einem Gasnetzabschnitt zur Verwendung für eine Gasabrechnung von Verbrauchern in dem Gasnetzabschnitt.

Figur 3 zeigt ein Blockdiagramm zur Illustration einer weiteren Ausführungsform eines Systems zur Bestimmung eines Brennwerts eines Brenngases in einem Gasnetzabschnitt zur Verwendung für eine Gasabrechnung von Verbrauchern in dem Gasnetzabschnitt.

Figur 4 zeigt ein Blockschaltbild eines Gasbrenners gemäß einer Ausführungsform eines Verbrauchers.

DETAILLIERTE BESCHREIBUNG DER FIGUREN UND BEVORZUGTER AUSFÜHRUNGSBEISPIELE

Im Folgenden werden Beispiele bzw. Ausführungsbeispiele der vorliegenden Erfindung detailliert unter Bezugnahme auf die beigefügten Figuren beschrieben. Gleiche bzw. ähnliche Elemente in den Figuren können hierbei mit gleichen Bezugszeichen bezeichnet sein, manchmal allerdings auch mit unterschiedlichen Bezugszeichen.

Es sei hervorgehoben, dass die vorliegende Erfindung jedoch in keiner Weise auf die im Folgenden beschriebenen Ausführungsbeispiele und deren Ausführungsmerkmale begrenzt bzw. eingeschränkt ist, sondern weiterhin Modifikationen der Ausführungsbeispiele umfasst, insbesondere diejenigen, die durch Modifikation der Merkmale der beschriebenen Beispiele bzw. durch Kombination einzelner oder mehrerer Merkmale der beschriebenen Beispiele im Rahmen des Schutzumfanges der Ansprüche umfasst sind.

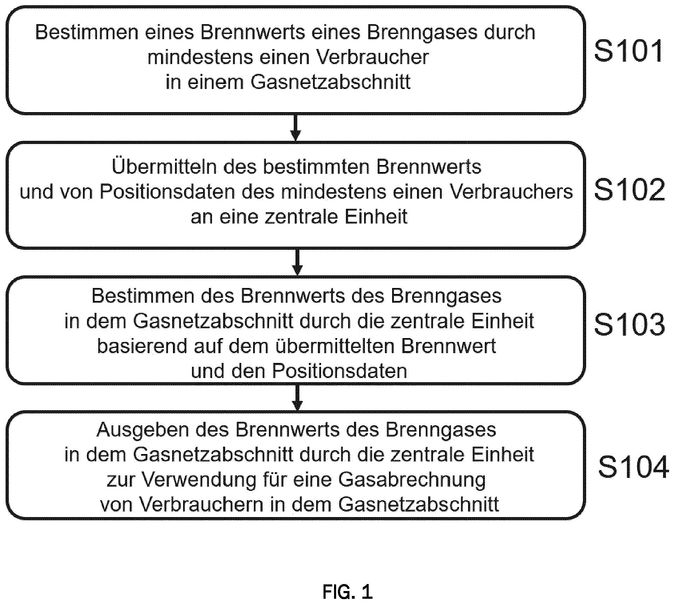

Figur 1 zeigt ein Flussdiagramm einer Ausführungsform eines Verfahrens zur Bestimmung eines Brennwerts eines Brenngases in einem Gasnetzabschnitt zur Verwendung für eine Gasabrechnung von Verbrauchern in dem Gasnetzabschnitt.

In Schritt S101 wird der Brennwert eines Brenngases in einem Gasnetzabschnitt durch mindestens einen Verbraucher bestimmt. Das Bestimmen des Brennwerts des Brenngases in Schritt S101 erfolgt dabei anhand von Regelungsparametern des Verbrauchers.

In einer bevorzugten Ausführungsform können in Schritt S101 zusätzlich auch Informationen zu einem Bestimmungszeitpunkt des Brennwerts, also beispielsweise einer Uhrzeit, durch den mindestens einen Verbraucher gespeichert werden.

Indem der Brennwert des Brenngases durch den mindestens einen Verbraucher bestimmt wird, kann durch das erfindungsgemäße Verfahren vorteilhaft auf zusätzliche Messpunkte, Messgeräte oder auch Umrüstungen, beispielsweise von Gaszählern, verzichtet werden. Das erfindungsgemäße Verfahren erlaubt damit eine einfache, sichere und verlässliche Bestimmung und effiziente Überwachung des Brennwerts des Brenngases in einem Gasnetzabschnitt.

Motiviert durch eine langfristige Einsparung an $CO_2$-Emissionen, ist die Einspeisung klimaneutral erzeugten Wasserstoffs als Zusatzgas im bestehenden Erdgasnetz mit Volumenanteilen von bis zu 40 Vol.-% geplant. Wasserstoff unterscheidet sich in seinen Eigenschaften dabei stark von Erdgas als Grundgas. Wasserstoff weist beispielsweise einen im Vergleich zu Erdgas volumenbezogen niedrigeren Brennwert auf. Das bedeutet, um eine vergleichbare Leistung zu erzielen, muss der Volumenstrom an Brenngas im Fall einer Wasserstoffbeimengung erhöht werden. Außerdem ist die Reaktionskinetik der Verbrennung gegenüber reinem Erdgas verändert, wodurch etwa die Flammengeschwindigkeit, -länge, und -geometrie, sowie die Flammentemperatur, die Zündeigenschaften und die Wärmeabstrahlung stark beeinflusst werden. So ist insbesondere die Bestimmung des Brennwerts des Brenngases (Grundgas oder Grundgas mit anteiligem Zusatzgas) durch den mindestens einen Verbraucher im Hinblick auf mögliche regionalspezifische und/oder zeitabhängige Schwankungen der Wasserstoffkonzentration im Gasnetzabschnitt und dem damit möglicherweise stark variierenden Brennwert des Brenngases von Bedeutung.

In einer bevorzugten Ausführungsform kann der Brennwert des Brenngases in Schritt S101 durch den mindestens einen Verbraucher anhand eines Zusammenhangs zwischen Modulationsgrad, Wärmebelastung und Luftmassenstrom bestimmt werden, wenn der mindestens eine Verbraucher ein Gasheizkessel ist.

Alternativ kann gemäß einer weiteren bevorzugten Ausführungsform, wenn der mindestens eine Verbraucher ein Gasheizkessel ist, in Schritt S101 der Brennwert des Brenngases auch anhand eines Zusammenhangs zwischen Wärmeleistung und Wirkungsgrad bestimmt werden.

In Schritt S102 werden dann der in Schritt S101 bestimmte Brennwert des Brenngases und die Positionsdaten des mindestens einen Verbrauchers von dem mindestens einen Verbraucher an eine zentrale Einheit übermittelt. In einer bevorzugten Ausführungsform kann die Übermittlung des in Schritt S101 bestimmten Brennwerts und der Positionsdaten des mindestens einen Verbrauchers an die zentrale Einheit in Schritt S102 in Echtzeit erfolgen. In einer überdies bevorzugten Ausführungsform können die Positionsdaten anonymisiert sein, also beispielsweise lediglich die Zuordnung eines Gasanschlusses zu einem Bereich des Gasnetzabschnitts umfassen. In einer weiteren bevorzugten Ausführungsform können zusätzlich in Schritt S102 auch die von dem mindestens einen Verbraucher gespeicherten Informationen zum Bestimmungszeitpunkt des Brennwerts des Brenngases an die zentrale Einheit übermittelt werden.

Das Verfahren umfasst weiterhin den Schritt S103 des Bestimmens des Brennwerts des Brenngases in dem Gasnetzabschnitt durch die zentrale Einheit basierend auf dem übermittelten Brennwert und den Positionsdaten. Dies kann insbesondere durch die Verbindung des durch den mindestens einen Verbraucher bestimmten Brennwerts des Brenngases mit den übermittelten Positionsdaten erfolgen. Bevorzugt kann in Schritt S103 der Brennwert des Brenngases in dem Gasnetzabschnitt durch die zentrale Einheit zusätzlich auch unter Berücksichtigung der Informationen zum Bestimmungszeitpunkt des Brennwerts des Brenngases durch den mindestens einen Verbraucher bestimmt werden. Auf diese Weise kann ein Zusammenhang zwischen dem Bestimmungszeitpunkt und dem Brennwert des Brenngases in dem Gasnetzabschnitt hergestellt und so eine zeitpunktgenaue Gasabrechnung ermöglicht werden.

In einer weiteren bevorzugten Ausführungsform können in der zentralen Einheit zusätzlich Informationen über eine Beschaffenheit des Gasnetzabschnitts gespeichert sein. Dann kann in Schritt S103 das Bestimmen des Brennwerts des Brenngases unter Berücksichtigung der Informationen über die Beschaffenheit des Gasnetzabschnitts erfolgen. Informationen über die Beschaffenheit des Gasnetzabschnitts können beispielsweise Informationen über die Gruppierung von Geräten, d.h. Verbrauchern in dem Gasnetzabschnitt sein.

Das Verfahren umfasst weiterhin den Schritt S104 des Ausgebens des in Schritt S103 bestimmten Brennwerts des Brenngases in dem Gasnetzabschnitt durch die zentrale Einheit zur Verwendung für eine Gasabrechnung von Verbrauchern in dem Gasnetzabschnitt.

Wie bereits dargelegt, kann die Zusammensetzung und damit auch der Brennwert eines in einem Gasnetzabschnitt geführten Brenngases zeitabhängig schwanken. In einer bevorzugten Ausführungsform kann daher Schritt S101 einmal innerhalb einer vorgegebenen Zeitspanne Δt durchgeführt werden. Gemäß einer weiteren bevorzugten Ausführungsform, kann Schritt S101 auch zwei oder mehrmals innerhalb der vorgegebenen Zeitspanne Δt durchgeführt werden. Die vorgegebene Zeitspanne Δt ist dabei erfindungsgemäß nicht eingeschränkt, kann aber beispielsweise 24h betragen.

In einer weiteren bevorzugten Ausführungsform kann der Brennwert des Brenngases in Schritt S101 jeweils durch zwei oder mehr Verbraucher bestimmt werden und in Schritt S102 die Brennwerte dann zusammen mit den zugehörigen Positionsdaten an die zentrale Einheit übermittelt werden.

In einer bevorzugten Ausführungsform kann die Übermittlung der in Schritt S101 bestimmten Brennwerte und der Positionsdaten der zwei oder mehr Verbraucher an die zentrale Einheit in Schritt S102 in Echtzeit erfolgen.

In einer überdies bevorzugten Ausführungsform können in Schritt S101 zusätzlich jeweils Informationen über den Bestimmungszeitpunkt des Brennwerts des Brenngases durch die zwei oder mehr Verbraucher gespeichert und in Schritt S102 an die zentrale Einheit übermittelt werden.

Aus den übermittelten Brennwerten und Positionsdaten und optional den Informationen über den Bestimmungszeitpunkt der Brennwerte kann dann in Schritt S103 ein gemittelter Brennwert des Brenngases in dem Gasnetzabschnitt durch die zentrale Einheit bestimmt werden.

In einer weiteren bevorzugten Ausführungsform kann der Brennwert des Brenngases in Schritt S101 durch die zwei oder mehr Verbraucher jeweils zeitgleich bestimmt werden. Alternativ kann, gemäß einer überdies bevorzugten Ausführungsform, der Brennwert des Brenngases durch die zwei oder mehr Verbraucher auch jeweils zeitversetzt bestimmt werden.

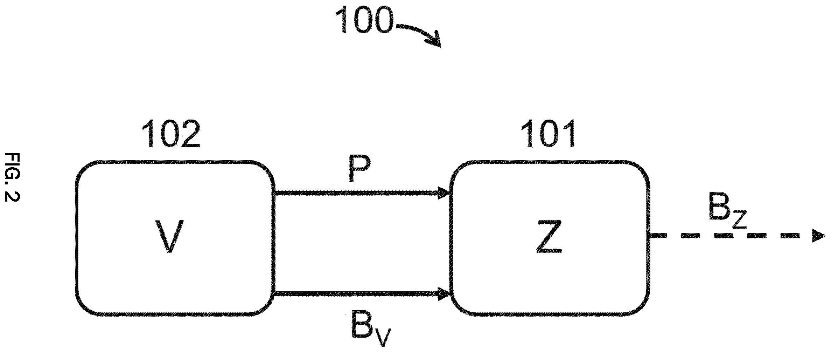

Figur 2 zeigt ein Blockdiagramm zur Illustration einer Ausführungsform eines Systems zur Bestimmung eines Brennwerts eines Brenngases in einem Gasnetzabschnitt zur Verwendung für eine Gasabrechnung von Verbrauchern in dem Gasnetzabschnitt.

Das System 100 umfasst einen Verbraucher (V) 102 und eine zentrale Einheit (Z) 101. Der Verbraucher 102 ist in einem der zentralen Einheit zugeordneten Gasnetzabschnitt angeordnet und weist eine Einrichtung zur Bestimmung eines Brennwerts eines Brenngases, sowie eine Einrichtung zur Übermittlung von Daten (IP-Schnittstelle) auf. Der Verbraucher 102 kann überdies auch eine Zeitmessvorrichtung aufweisen, um beispielsweise zeitliche Intervalle für die Bestimmung des Brennwerts festzulegen. Der Verbraucher 102 kann zusätzlich auch eine Messsensorik aufweisen.

Mittels der Einrichtung zur Bestimmung des Brennwerts wird durch den Verbraucher 102 ein Brennwert Bv des Brenngases bestimmt. Der Brennwert Bv wird gemeinsam mit den Positionsdaten P des Verbrauchers 102 an die zentrale Einheit 101 übermittelt. Die zentrale Einheit 101 bestimmt dann anhand des Brennwerts Bv und der Positionsdaten P den Brennwert Bz des Brenngases in dem Gasnetzabschnitt, in welchem der Verbraucher 102 angeordnet ist. Die zentrale Einheit 101 gibt dann den Brennwert des Brenngases Bz zur Verwendung für eine Gasabrechnung der Verbraucher in dem Gasnetzabschnitt aus. Dies kann beispielsweise über eine Push-Mitteilung an die Verbraucher in dem Gasnetzabschnitt oder den jeweiligen Gasversorger erfolgen oder auch durch eine Bereitstellung des ermittelten Brennwerts zum Abruf durch die Verbraucher in dem Gasnetzabschnitt oder den jeweiligen Gasversorger.

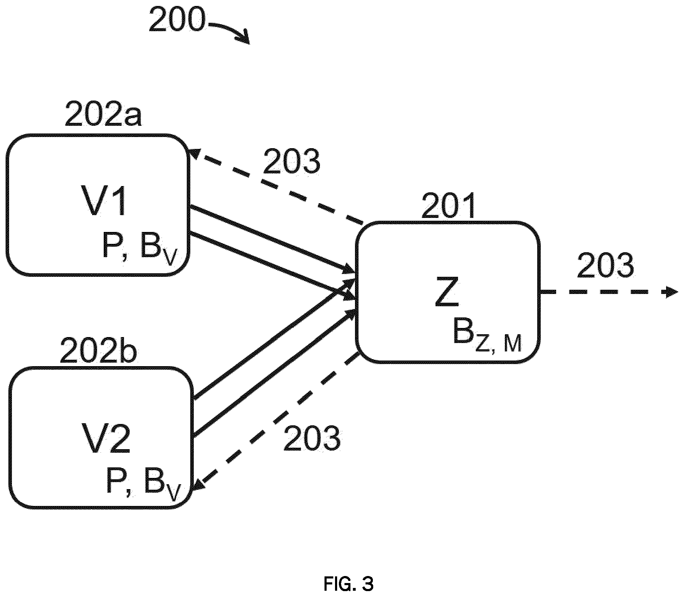

Figur 3 zeigt ein Blockdiagramm zur Illustration einer weiteren Ausführungsform eines Systems zur Bestimmung eines Brennwerts eines Brenngases in einem Gasnetzabschnitt zur Verwendung für eine Gasabrechnung von Verbrauchern in dem Gasnetzabschnitt.

Im Unterschied zu dem in Figur 2 gezeigten System 100, weist das in Figur 3 gezeigte System 200 zwei Verbraucher V1, 202a und V2, 202b mit jeweils einer Einrichtung zur Bestimmung eines Brennwerts eines Brenngases und einer Einrichtung zur Übermittlung von Daten auf. Hier sei angemerkt, dass das System nicht auf zwei Verbraucher beschränkt ist, die Anzahl an Verbrauchern wird von der Ausgestaltung des jeweiligen Gasnetzabschnitts abhängen.

Wie in Figur 3 gezeigt, wird jeweils ein Brennwert Bv durch die zwei Verbraucher 202a und 202b bestimmt und gemeinsam mit Positionsdaten P an die zentrale Einheit 201 übermittelt. Die zentrale Einheit 201 bestimmt dann einen gemittelten Brennwert des Brenngases $B_{Z,M}$ aus den erhaltenen, individuell bestimmten Brennwerten Bv in Verbindung mit den jeweiligen Positionsdaten P. In einer bevorzugten Ausführungsform kann überdies der gemittelte Brennwert $B_{Z,M}$ auch unter Berücksichtigung von in der zentralen Einheit 201 gespeicherten Informationen über eine Beschaffenheit des Gasnetzabschnitts erfolgen.

Der gemittelte Brennwert $B_{Z,M}$ wird dann von der zentralen Einheit 201 zur Verwendung für eine Gasabrechnung der Verbraucher in dem Gasnetzabschnitt ausgegeben 203.

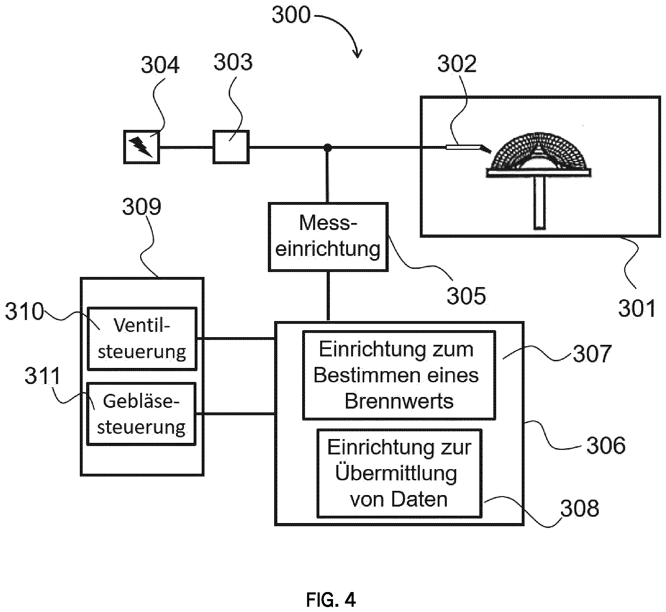

Figur 4 zeigt ein Blockschaltbild eines Gasbrenners gemäß einer Ausführungsform eines Verbrauchers.

Der Gasbrenner 300 besitzt eine Brennkammer 301 in der unter Zuführung eines Luft-Brenngas-Gemisches ein Verbrennungsvorgang ablaufen kann. In die Brennkammer 301 ragt eine Zündelektrode 302 hinein. Optional kann auch eine Ionisationselektrode in dem Gasbrenner 300 vorgesehen sein, die zusätzlich in die Brennkammer hineinragt. Eine Ionisationselektrode wird im Allgemeinen zur Flammenüberwachung eingesetzt.

Die Zündelektrode 302 steht mit einer Einrichtung zur Erzeugung einer Zündspannung 304 so in Verbindung, dass die Zündelektrode 302 von der Einrichtung zur Erzeugung der Zündspannung 304 getrennt werden kann. Dies kann durch eine zwischen der Zündelektrode 302 und der Einrichtung zur Erzeugung der Zündspannung 304 geschaltete Schalteranordnung 303 geschehen. Dabei kann die Schalteranordnung 303 insbesondere so eingerichtet sein, dass nach einer Trennung der Zündelektrode 302 von der Einrichtung zur Erzeugung der Zündspannung 304 die Zündelektrode 302 als eine passive Elektrode geschaltet ist.

Der Gasbrenner 300 weist außerdem eine Messeinrichtung 305 auf. Über die Messeinrichtung 305 kann beispielsweise unter Ausnutzung des glühelektrische Effekts auf die Temperatur der Flamme an der Elektrode rückgeschlossen werden und so der Flammentemperaturverlauf bei entsprechend eingestelltem Lastwert gemessen werden. Hierzu steht die Schalteranordnung 303 mit der Messeinrichtung 305 in Verbindung und kann Signale von der Messeinrichtung 305 empfangen.

Die Messeinrichtung 305 steht weiterhin mit einer Regelschaltung 306 in Verbindung. Über die Regelschaltung 306 kann mittels einer Brennersteuerung 309 die Verbrennung im Gasbrenner 300 geregelt werden. Die Brennersteuerung 309 weist hierzu entsprechend eine Ventilsteuerung 310 zur Veränderung des Brenngasanteils im Luft-Brenngas-Gemisch auf, sowie eine Gebläsesteuerung 311 zur Variation des Luftanteils.

Die Regelschaltung 306 weist insbesondere eine Einrichtung zum Bestimmen eines Brennwerts 307 auf. Die Einrichtung zum Bestimmen des Brennwerts 307 ist mit der Messeinrichtung 305 verbunden und empfängt die ermittelten Messwerte zur Bestimmung der Flammentemperaturverläufe, welche mittels der Zündelektrode 302 in der Brennkammer 301 ermittelt wurden. In der Einrichtung zum Bestimmen des Brennwerts 307 werden die Flammentemperaturverläufe ausgewertet und der Brennwert des Brenngases bestimmt.

Basierend auf dem bestimmten Brennwert kann in der Regelschaltung 306 eine Regelgröße für die Regelung der Verbrennung im Gasbrenner 300 abgeleitet werden. Die Regelgröße kann beispielsweise eine angepasste Luftzahl $\lambda$ sein und/oder ein veränderter Volumenstrom. Durch die Beimengung von Wasserstoff verschiebt sich beispielsweise das Maximum der laminaren Flammengeschwindigkeit zu kleineren Lambda Werten, außerdem wird ein höherer Volumenstrom für eine mit reinem Erdgas vergleichbare Leistung benötigt.

Die Regelgröße wird dann durch die Regelschaltung 306 an die Brennersteuerung 309 übermittelt, welche dann über die Ventilsteuerung 310 und/oder die Gebläsesteuerung 311 die Verbrennung im Gasbrenner 300 entsprechend regeln bzw. an das veränderte Brenngas anpassen kann.

Die Einrichtung zur Bestimmung des Brennwerts 307 ist überdies mit einer Einrichtung zur Übermittlung von Daten 308 verbunden, mittels welcher der bestimmte Brennwert und Positionsdaten des Gasbrenners 300 an eine zentrale Einheit, beispielsweise eine Cloud, übermittelt werden können. Die Einrichtung zur Übermittlung von Daten 308 kann überdies auch Daten von der Cloud empfangen.

BEZUGSZEICHEN

| | |
|---|---|
| 100 | System zur Bestimmung eines Brennwerts eines Brenngases |
| 101 | zentrale Einheit |
| 102 | Verbraucher |
| 200 | System zur Bestimmung eines Brennwerts eines Brenngases |
| 201 | zentrale Einheit |
| 202a | Erster Verbraucher |
| 202b | Zweiter Verbraucher |
| 300 | Gasbrenner |
| 301 | Brennkammer |
| 302 | Zündelektrode |
| 303 | Schalteranordnung |
| 304 | Einrichtung zur Erzeugung einer Zündspannung |
| 305 | Messeinrichtung |
| 306 | Regelschaltung |
| 307 | Einrichtung zum Bestimmen eines Brennwerts eines Brenngases |
| 308 | Einrichtung zur Übermittlung von Daten |
| 309 | Brennersteuerung |
| 310 | Ventilsteuerung |
| 311 | Gebläsesteuerung |
| V | Verbraucher |
| P | Positionsdaten |
| $B_V$ | durch Verbraucher bestimmter Brennwert des Brenngases |
| Bz | durch zentrale Einheit bestimmter Brennwert des Brenngases im Gasnetzabschnitt |
| $B_{Z,M}$ | durch zentrale Einheit bestimmter gemittelter Brennwert des Brenngases im Gasnetzabschnitt |

**Patentansprüche**

1. Verfahren zur Bestimmung eines Brennwerts eines Brenngases in einem Gasnetzabschnitt zur Verwendung für eine Gasabrechnung von Verbrauchern (102, 202a, 202b, 300) in dem Gasnetzabschnitt, wobei das Verfahren umfasst:

   a) Bestimmen (S101) eines Brennwerts eines Brenngases (Bv) durch mindestens einen Verbraucher (102, 202a, 202b, 300) in dem Gasnetzabschnitt;

b) Übermitteln (S102) des in Schritt a) bestimmten Brennwerts ($B_V$) und von Positionsdaten (P) des mindestens einen Verbrauchers (102, 202a, 202b, 300) an eine zentrale Einheit (101, 201);

c) Bestimmen (S103) des Brennwerts des Brenngases in dem Gasnetzabschnitt (Bz) durch die zentrale Einheit (101, 201) basierend auf dem übermittelten Brennwert (Bv) und den Positionsdaten (P); und

d) Ausgeben (S104) des Brennwerts des Brenngases in dem Gasnetzabschnitt (Bz) durch die zentrale Einheit (101, 201) zur Verwendung für eine Gasabrechnung von Verbrauchern (102, 202a, 202b, 300) in dem Gasnetzabschnitt,

**dadurch gekennzeichnet, dass**

der mindestens eine Verbraucher (102, 202a, 202b, 300) ein Gasheizkessel ist und in Schritt a) der Brennwert des Brenngases (Bv) durch den mindestens einen Verbraucher (102, 202a, 202b) anhand eines Zusammenhangs zwischen Modulationsgrad, Wärmebelastung und Luftmassenstrom oder anhand eines Zusammenhangs zwischen Wärmeleistung und Wirkungsgrad bestimmt wird.

2. Verfahren nach Anspruch 1, **dadurch gekennzeichnet, dass** in der zentralen Einheit (101, 201) zusätzlich Informationen über eine Beschaffenheit des Gasnetzabschnitts gespeichert sind und in Schritt c) das Bestimmen des Brennwerts des Brenngases (Bz) unter Berücksichtigung der Informationen über die Beschaffenheit des Gasnetzabschnitts erfolgt.

3. Verfahren nach Anspruch 1 oder 2, **dadurch gekennzeichnet, dass** die Positionsdaten (P) des mindestens einen Verbrauchers (102, 202a, 202b, 300) anonymisierte Positionsdaten sind.

4. Verfahren nach einem der Ansprüche 1 bis 3, **dadurch gekennzeichnet, dass** in Schritt b) der in Schritt a) bestimmte Brennwert (Bv) und die Positionsdaten (P) des mindestens einen Verbrauchers (102, 202a, 202b, 300) in Echtzeit an die zentrale Einheit (101, 201) übermittelt werden.

5. Verfahren nach einem der Ansprüche 1 bis 4, **dadurch gekennzeichnet, dass** in Schritt a) zusätzlich Informationen über einen Bestimmungszeitpunkt des Brennwerts des Brenngases (Bv) durch den mindestens einen Verbraucher (102, 202a, 202b, 300) gespeichert werden und in Schritt b) an die zentrale Einheit übermittelt werden.

6. Verfahren nach zumindest einem der Ansprüche 1 bis 5, **dadurch gekennzeichnet, dass** Schritt a) mindestens einmal innerhalb einer vorgegebenen Zeitspanne $\Delta t$ durchgeführt wird.

7. Verfahren nach Anspruch 6, **dadurch gekennzeichnet, dass** Schritt a) zwei oder mehrmals innerhalb der vorgegebenen Zeitspanne $\Delta t$ durchgeführt wird.

8. Verfahren nach zumindest einem der Ansprüche 1 bis 7, **dadurch gekennzeichnet, dass** in Schritt a) der Brennwert des Brenngases (Bv) jeweils durch zwei oder mehr Verbraucher (102, 202a, 202b, 300) bestimmt wird, wobei in Schritt b) die in Schritt a) bestimmten Brennwerte (Bv) und die Positionsdaten (P) der zwei oder mehr Verbraucher (102, 202a, 202b, 300) an die zentrale Einheit (101, 201) übermittelt werden und in Schritt c) ein gemittelter Brennwert des Brenngases in dem Gasnetzabschnitt ($B_{Z,M}$) durch die zentrale Einheit (101, 201) bestimmt wird.

9. Verfahren nach Anspruch 8, **dadurch gekennzeichnet, dass** in Schritt a) der Brennwert des Brenngases (Bv) durch die zwei oder mehr Verbraucher (102, 202a, 202b, 300) jeweils zeitgleich bestimmt wird.

10. Verfahren nach Anspruch 8, **dadurch gekennzeichnet, dass** in Schritt a) der Brennwert des Brenngases (Bv) durch die zwei oder mehr Verbraucher (102, 202a, 202b, 300) jeweils zeitversetzt bestimmt wird.

11. System (100, 200) zur Bestimmung eines Brennwerts eines Brenngases in einem Gasnetzabschnitt zur Verwendung für eine Gasabrechnung von Verbrauchern (102, 202a, 202b, 300) in dem Gasnetzabschnitt, wobei das System umfasst:

- einen Gasnetzabschnitt, in welchem mindestens ein Verbraucher (102, 202a, 202b, 300) angeordnet ist, wobei der mindestens eine Verbraucher (102, 202a, 202b, 300) eine Einrichtung zur Übermittlung von Daten (308) aufweist und eingerichtet ist, einen Brennwert eines Brenngases (Bv) zu bestimmen (307); und

- eine zentrale Einheit (101, 201), **dadurch gekennzeichnet, dass** die zentrale Einheit (101, 201) und der mindestens eine Verbraucher (102, 202a, 202b, 300) dazu eingerichtet sind, um zu bewirken, dass das System (100, ein Verfahren nach zumindest einem der Ansprüche 1 bis 8 durchführt.

**12.** System (100, 200) nach Anspruch 11, **dadurch gekennzeichnet, dass** der mindestens eine Verbraucher (102, 202a, 202b, 300) zusätzlich eine Zeitmessvorrichtung aufweist.

**13.** System (100, 200) nach Anspruch 11 oder 12, **dadurch gekennzeichnet, dass** in dem Gasnetzabschnitt zwei oder mehr Verbraucher (102, 202a, 202b, 300) angeordnet sind, wobei die zwei oder mehr Verbraucher (102, 202a, 202b, 300) eine Einrichtung zur Übermittlung von Daten (308) aufweisen und jeweils eingerichtet sind, einen Brennwert eines Brenngases (Bv) zu bestimmen (307).

**Claims**

**1.** A method for determining a calorific value of a fuel gas in a gas network section for use for gas billing of consumers (102, 202a, 202b, 300) in the gas network section, the method comprising:

a) determining (S101) a calorific value of a fuel gas (Bv) by at least one consumer (102, 202a, 202b, 300) in the gas network section;
b) transmitting (S102) the calorific value (Bv) determined in step a) and position data (P) of the at least one consumer (102, 202a, 202b, 300) to a central unit (101, 201);
c) determining (S103) the calorific value of the fuel gas in the gas network section ($B_Z$) by the central unit (101, 201) based on the transmitted calorific value ($B_V$) and the position data (P); and
d) outputting (S104) the calorific value of the fuel gas in the gas network section (Bz) by the central unit (101, 201) for use for gas billing of consumers (102, 202a, 202b, 300) in the gas network section,

**characterized in that**
the at least one consumer (102, 202a, 202b, 300) is a gas heating boiler and in step a) the calorific value of the fuel gas ($B_V$) is determined by the at least one consumer (102, 202a, 202b) on the basis of a relationship between degree of modulation, thermal load and air mass flow or on the basis of a relationship between thermal power and efficiency.

**2.** The method according to claim 1, **characterized in that** information about a nature of the gas network section is additionally stored in the central unit (101, 201) and in step c) the calorific value of the fuel gas (Bz) is determined taking into account the information about the nature of the gas network section.

**3.** The method according to claim 1 or 2, **characterized in that** the position data (P) of the at least one consumer (102, 202a, 202b, 300) are anonymized position data.

**4.** The method according to one of claims 1 to 3, **characterized in that** in step b) the calorific value ($B_V$) determined in step a) and the position data (P) of the at least one consumer (102, 202a, 202b, 300) are transmitted in real time to the central unit (101, 201).

**5.** The method according to one of claims 1 to 4, **characterized in that** in step a) information about a determination time of the calorific value of the fuel gas (Bv) by the at least one consumer (102, 202a, 202b, 300) is additionally stored and in step b) is transmitted to the central unit.

**6.** The method according to at least one of claims 1 to 5, **characterized in that** step a) is carried out at least once within a predetermined time span $\Delta t$.

**7.** The method according to claim 6, **characterized in that** step a) is carried out two or more times within the predetermined time span $\Delta t$.

**8.** The method according to at least one of claims 1 to 7, **characterized in that** in step a) the calorific value of the fuel gas ($B_V$) is respectively determined by two or more consumers (102, 202a, 202b, 300), wherein in step b) the calorific values ($B_V$) determined in step a) and the position data (P) of the two or more consumers (102, 202a, 202b, 300) are transmitted to the central unit (101, 201) and in step c) an averaged calorific value of the fuel gas in the gas network section ($B_{Z, M}$) is determined by the central unit (101, 201).

**9.** The method according to claim 8, **characterized in that** in step a) the calorific value of the fuel gas ($B_V$) is respectively determined simultaneously by the two or more consumers (102, 202a, 202b, 300).

**10.** The method according to claim 8, **characterized in that** in step a) the calorific value of the fuel gas (B$_V$) is respectively determined with a time offset by the two or more consumers (102, 202a, 202b, 300).

**11.** A system (100, 200) for determining a calorific value of a fuel gas in a gas network section for use for gas billing of consumers (102, 202a, 202b, 300) in the gas network section, the system comprising:

- a gas network section in which at least one consumer (102, 202a, 202b, 300) is arranged, wherein the at least one consumer (102, 202a, 202b, 300) has a device for transmitting data (308) and is configured to determine (307) a calorific value of a fuel gas (Bv); and
- a central unit (101, 201),

**characterized in that** the central unit (101, 201) and the at least one consumer (102, 202a, 202b, 300) are configured to cause the system (100) to carry out a method according to at least one of claims 1 to 8.

**12.** The system (100, 200) according to claim 11, **characterized in that** the at least one consumer (102, 202a, 202b, 300) additionally has a time measuring device.

**13.** The system (100, 200) according to claim 11 or 12, **characterized in that** two or more consumers (102, 202a, 202b, 300) are arranged in the gas network section, wherein the two or more consumers (102, 202a, 202b, 300) have a device for transmitting data (308) and are respectively configured to determine (307) a calorific value of a fuel gas (Bv).

**Revendications**

**1.** Procédé de détermination d'une valeur calorifique d'un gaz combustible dans une section de réseau de gaz à utiliser pour une facturation de gaz de consommateurs (102, 202a, 202b, 300) dans la section de réseau de gaz, le procédé comprenant :

a) la détermination (S101) d'une valeur calorifique d'un gaz combustible (Bv) par au moins un consommateur (102, 202a, 202b, 300) dans la section de réseau de gaz ;
b) la transmission (S102) de la valeur calorifique (B$_V$) déterminée à l'étape a) et de données de position (P) de l'au moins un consommateur (102, 202a, 202b, 300) à une unité centrale (101, 201) ;
c) la détermination (S103) de la valeur calorifique du gaz combustible dans la section de réseau de gaz (B$_Z$) par l'unité centrale (101, 201) sur la base de la valeur calorifique transmise (B$_V$) et des données de position (P) ; et
d) la sortie (S104) de la valeur calorifique du gaz combustible dans la section de réseau de gaz (Bz) par l'unité centrale (101, 201) à utiliser pour une facturation de gaz de consommateurs (102, 202a, 202b, 300) dans la section de réseau de gaz,

**caractérisé en ce que**
l'au moins un consommateur (102, 202a, 202b, 300) est une chaudière à gaz et à l'étape a), la valeur calorifique du gaz combustible (B$_V$) est déterminée par l'au moins un consommateur (102, 202a, 202b) à l'aide d'une relation entre le degré de modulation, la charge thermique et le débit massique d'air ou à l'aide d'une relation entre la puissance thermique et le rendement.

**2.** Procédé selon la revendication 1, **caractérisé en ce que** des informations sur une nature de la section de réseau de gaz sont en outre stockées dans l'unité centrale (101, 201) et à l'étape c), la détermination de la valeur calorifique du gaz combustible (Bz) est effectuée en tenant compte des informations sur la nature de la section de réseau de gaz.

**3.** Procédé selon la revendication 1 ou 2, **caractérisé en ce que** les données de position (P) de l'au moins un consommateur (102, 202a, 202b, 300) sont des données de position anonymisées.

**4.** Procédé selon l'une quelconque des revendications 1 à 3, **caractérisé en ce qu'**à l'étape b), la valeur calorifique (B$_V$) déterminée à l'étape a) et les données de position (P) de l'au moins un consommateur (102, 202a, 202b, 300) sont transmises en temps réel à l'unité centrale (101, 201).

**5.** Procédé selon l'une quelconque des revendications 1 à 4, **caractérisé en ce qu'**à l'étape a), des informations sur un instant de détermination de la valeur calorifique du gaz combustible (B$_V$) par l'au moins un consommateur (102, 202a, 202b, 300) sont en outre stockées et sont transmises à l'étape b) à l'unité centrale.

**6.** Procédé selon au moins l'une quelconque des revendications 1 à 5, **caractérisé en ce que** l'étape a) est réalisée au moins une fois dans un intervalle de temps prédéfini $\Delta t$.

**7.** Procédé selon la revendication 6, **caractérisé en ce que** l'étape a) est réalisée deux ou plusieurs fois dans l'intervalle de temps prédéfini $\Delta t$.

**8.** Procédé selon au moins l'une quelconque des revendications 1 à 7, **caractérisé en ce qu'**à l'étape a), la valeur calorifique du gaz combustible ($B_V$) est déterminée respectivement par deux consommateurs ou plus (102, 202a, 202b, 300), à l'étape b), les valeurs calorifiques ($B_V$) déterminées à l'étape a) et les données de position (P) des deux consommateurs ou plus (102, 202a, 202b, 300) étant transmises à l'unité centrale (101, 201) et à l'étape c), une valeur calorifique moyenne du gaz combustible dans la section de réseau de gaz ($B_{Z,\,M}$) étant déterminée par l'unité centrale (101, 201).

**9.** Procédé selon la revendication 8, **caractérisé en ce qu'**à l'étape a), la valeur calorifique du gaz combustible ($B_V$) est déterminée respectivement en même temps par les deux consommateurs ou plus (102, 202a, 202b, 300).

**10.** Procédé selon la revendication 8, **caractérisé en ce qu'**à l'étape a), la valeur calorifique du gaz combustible ($B_V$) est déterminée respectivement de manière décalée dans le temps par les deux consommateurs ou plus (102, 202a, 202b, 300).

**11.** Système (100, 200) de détermination d'une valeur calorifique d'un gaz combustible dans une section de réseau de gaz à utiliser pour une facturation de gaz de consommateurs (102, 202a, 202b, 300) dans la section de réseau de gaz, le système comprenant :

- une section de réseau de gaz dans laquelle est disposé au moins un consommateur (102, 202a, 202b, 300), l'au moins un consommateur (102, 202a, 202b, 300) présentant un dispositif de transmission de données (308) et étant conçu pour déterminer (307) une valeur calorifique d'un gaz combustible (Bv) ; et
- une unité centrale (101, 201),

**caractérisé en ce que** l'unité centrale (101, 201) et l'au moins un consommateur (102, 202a, 202b, 300) sont conçus pour amener le système (100) à réaliser un procédé selon au moins l'une quelconque des revendications 1 à 8.

**12.** Système (100, 200) selon la revendication 11, **caractérisé en ce que** l'au moins un consommateur (102, 202a, 202b, 300) présente en outre un dispositif de mesure du temps.

**13.** Système (100, 200) selon la revendication 11 ou 12, **caractérisé en ce que** deux consommateurs ou plus (102, 202a, 202b, 300) sont disposés dans la section de réseau de gaz, les deux consommateurs ou plus (102, 202a, 202b, 300) présentant un dispositif de transmission de données (308) et étant respectivement conçus pour déterminer (307) une valeur calorifique d'un gaz combustible (Bv).

Bestimmen eines Brennwerts eines Brenngases durch
mindestens einen Verbraucher
in einem Gasnetzabschnitt

S101

Übermitteln des bestimmten Brennwerts
und von Positionsdaten des mindestens einen Verbrauchers
an eine zentrale Einheit

S102

Bestimmen des Brennwerts des Brenngases
in dem Gasnetzabschnitt durch die zentrale Einheit
basierend auf dem übermittelten Brennwert
und den Positionsdaten

S103

Ausgeben des Brennwerts des Brenngases
in dem Gasnetzabschnitt durch die zentrale Einheit
zur Verwendung für eine Gasabrechnung
von Verbrauchern in dem Gasnetzabschnitt

S104

FIG. 1

FIG. 2

FIG. 3

FIG. 4

**IN DER BESCHREIBUNG AUFGEFÜHRTE DOKUMENTE**

**In der Beschreibung aufgeführte Patentdokumente**

- EP 2450704 B1
- EP 3287750 B1
- DE 102018106576 A1
- FR 3030034 A1
- DE 102019115973 A1
- WO 2004065915 A1